# EUROPEAN PATENT SPECIFICATION

(11) **EP 4 175 633 B1**
(45) Date of publication and mention of the grant of the patent: **29.10.2025**
(21) Application number: 21740607.3
(22) Date of filing: 02.07.2021
(51) Int. Cl.: A61K 31/4162, A61K 31/4439, A61K 31/497, A61P 11/00, A61P 35/00

(54) **THIENO[3,4-C]PYRAZOL-3-YL ACETAMIDES AS AUTOTAXIN INHIBITORS**
THIENO[3,4-C]PYRAZOL-3-YL ACETAMIDE ALS AUTOTAXIN-INHIBITOREN
THIÉNO[3,4-C]PYRAZOL-3-YL ACÉTAMIDES UTILISÉS EN TANT QU'INHIBITEURS DE L'AUTOTAXINE

(30) Priority: 02.07.2020 GR 20200100391; 24.06.2021 GR 20210100422
(43) Date of publication of application: 10.05.2023
(73) Proprietor: Uni-Pharma Kleon Tsetis Pharmaceutical Laboratories S.A., 14564 Kifissia, Attica (GR); B.S.R.C. "Alexander Fleming", 166 72 Vari Attica (GR)
(72) Inventor: AIDINIS, Vassilios, 163 46 Hlioupoli (GR); MATRALIS, Alexios, 174 55 Kalamaki Attica (GR)
(74) Representative: Sonnenhauser, Thomas Martin
(86) International application number: PCT/GR2021/000045
(87) International publication number: WO 2022/003377

(56) References cited:
- EP-A1- 1 698 335
- US-A1- 2008 081 833
- US-A1- 2010 075 993
- US-A1- 2010 087 415
- MILLER SIMON ET AL: "Isoform-selective regulation of mammalian cryptochromes", NATURE CHEMICAL BIOLOGY, NATURE PUB. GROUP, NEW YORK, vol. 16, no. 6, 30 March 2020 (2020-03-30), pages 676 - 685, XP037145347, ISSN: 1552-4450, [retrieved on 20200330], DOI: 10.1038/S41589-020-0505-1
- CASTAGNA DIANA ET AL: "Development of Autotaxin Inhibitors: An Overview of the Patent and Primary Literature : Miniperspective", JOURNAL OF MEDICINAL CHEMISTRY, vol. 59, no. 12, 28 January 2016 (2016-01-28), US, pages 5604 - 5621, XP055793567, ISSN: 0022-2623, DOI: 10.1021/acs.jmedchem.5b01599
- MAHER TOBY M ET AL: "Trial registration numbers", BMJ OPEN RESPIRATORY RESEARCH, vol. 6, no. 1, 1 May 2019 (2019-05-01), pages e000422, XP055793568, DOI: 10.1136/bmjresp-2019-000422
- MAGKRIOTI CHRISTIANA ET AL: "Structure-Based Discovery of Novel Chemical Classes of Autotaxin Inhibitors", INTERNATIONAL JOURNAL OF MOLECULAR SCIENCES, vol. 21, no. 19, 23 September 2020 (2020-09-23), pages 7002, XP055793531, DOI: 10.3390/ijms21197002

## Description

The present invention relates to pharmaceutical compounds according to formula (A) or their pharmaceutically acceptable salts, wherein R₁ and R₂ substituents are fully defined. Chemical compounds according to formula (A) exhibit autotaxin inhibition activity and may be used in the treatment of pulmonary fibrosis, pulmonary allograft fibrosis, hepatic fibrosis, renal fibrosis, cardiac fibrosis, pneumonia, Covid-19 disease, bacterial diseases, neurodegenerative diseases**,** diabetes or diabetes mellitus, metabolic syndrome diseases and cancer.

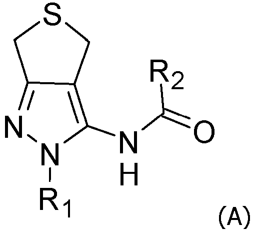

Autotaxin (ATX) was first isolated in the mid-90s as an autocrine motility factor for melanoma cells [Stracke, M.L., et al., Identification, purification, and partial sequence analysis of autotaxin, a novel motility-stimulating protein. J Biol Chem, 1992, 267(4): p. 2524-9]. Later, ATX was recognized as a member of ecto-nucleotide pyrophosphatases and phosphodiesterases (NPPs) family, which are extracellular enzymes that hydrolyze phosphodiester bonds of nucleotides and their derivatives and renamed as ENPP2. Even later, it was found that ATX acts mainly as phospholipase D, catalyzing the hydrolysis of extracellular lysophosphatidylcholine (LPC) to lysophosphatidic acid (LPA) [Tokumura, A., et al., Identification of human plasma lysophospholipase D, a lysophosphatidic acid-producing enzyme, as autotaxin, a multifunctional phosphodiesterase. J Biol Chem, 2002, 277(42): p. 39436-42]. LPA is a simple lipid consisting of a glycerol chain bearing one hydroxyl-group, a phosphate group and a long chain saturated or unsaturated fatty acid-group. LPA is detected in various biological fluids including serum, plasma, activated platelets and saliva. LPA acts as a phospholipid growth factor and affects almost all cell types, while it is also involved in pathological conditions including cancer, atherosclerosis and wound healing.

Increased ATX expression in tissues has been detected, including adipose tissues, brain, lung, spinal cord, ovaries, testes, intestine, kidneys, and lymph nodes. ATX is also found in many biological fluids such as serum, plasma, alveolar secretion, pus fluid, cerebrospinal fluid, peritoneal fluid, synovial fluid and urine. ATX plays a crucial role in embryonic cell growth, in the development of central nervous system and lipogenesis [Fotopoulou S, et al. ATX expression and LPA signaling are vital for the development of the nervous system. Dev. Biol. 2010; 339(2):451-64]. On the contrary, ATX expression in adult models proved to be unnecessary [Katsifa A, et al. The bulk of Autotaxin activity is dispensable for adult mouse Life. PLoS One. 2015; 10(11):e0143083]. Moreover, ATX expression has been detected in a number of chronic inflammatory diseases and different, while still upregulated, levels of ATX have been reported in various types of cancer [Barbayianni, E., et al., Autotaxin, a secreted lysophospholipase D, as a promising therapeutic target in chronic inflammation and cancer. Prog Lipid Res., 2015, 58: p. 76-96]. Beyond the melanoma cells, ATX is also detected in various forms of cancer cells, furthermore, plays an important role in cancer invasion and metastasis through the motility increase of cancer cells and the induction of cancer angiogenesis [Leblanc, R., & Peyruchaud, O. New insights into the autotaxin/LPA axis in cancer development and metastasis. Experimental Cell Research, 2015. 333(2): p. 183-189]. It is reported that ATX is overexpressed in several cancers including breast cancer as well as non-small-cell lung cancer, with this overexpression being related to the infiltration ability of cancer cells. Additionally, ATX is found highly upregulated in glioblastoma, thyroid and renal cancer, B-cell and Hodgkin's lymphomas and in glioblastoma multiforme.

It is already mentioned that the ATX / LPA pathway plays a key role in several pathological and inflammatory conditions. In this context, upregulated levels of ATX and LPA were detected in the joints of patients with rheumatoid arthritis. Increased levels of ATX and LPA signaling have been found to promote joint hyperplasia and progressive cartilage damage [Orosa, B., et al. The autotaxin-lysophosphatidic acid pathway in pathogenesis of rheumatoid arthritis. European Journal of Pharmacology 2015. 765: p. 228-233].

Other studies have shown that chronic inflammation of the liver stimulates the secretion of ATX by hepatocytes, while LPA appears to activate liver cells and enhance fibroproliferative pathways [Watanabe, N., et al., Both plasma lysophosphatidic acid and serum autotaxin levels are increased in chronic hepatitis C. J Clin Gastroenterol, 2007. 41 (6): p. 616-23]. ATX serum levels represent an important indicator of fibrosis prognosis in patients with chronic hepatitis B and C, as well as the most reliable indicator for determining the stages of fibrosis. Therefore, ATX can be used as a diagnostic and pharmaceutical tool to inhibit the progression of viral hepatitis to cancer.

ATX also plays a critical role in diseases related to central nervous system, where increased levels of ATX and LPA have been reported in serum and cerebrospinal fluid of patients with multiple sclerosis [Dennis, J., et al., Phosphodiesterase-Iα/autotaxin (PD-Iα/ATX): a multifunctional protein involved in central nervous system development and disease. J Neurosci Res, 2005. 82(6): p. 737-42]. Furthermore, high ATX levels are associated with greater danger for mild cognitive impairment or Alzheimer disease [McLimans, K. E. & Willette, A. A. Autotaxin is related to metabolic dysfunction and predicts Alzheimer's disease outcomes. Journal of Alzheimer's disease 2017. 56: p. 403-413].

Several clinical trials in animal models as well as in fibrotic lungs of patients with idiopathic pulmonary fibrosis showed increased levels of ATX in the alveolar space [Ninou, I., et al. Autotaxin in pathophysiology and pulmonary fibrosis. Frontiers in Medicine 2018. 5: p. 180]. LPA has also been reported to induce epithelial cell apoptosis in a model of pulmonary fibrosis. These cells are known to express cytokines such as tumor necrosis factor-α (TNF-α), which is involved in the pathogenesis of pulmonary fibrosis [Oikonomou, N. et al. Pulmonary autotaxin expression contributes to the pathogenesis of pulmonary fibrosis. American Journal of Respiratory Cell and Molecular Biology 2012. 47, 566-574]. Finally, elevated ATX levels have also been associated with asthma pathogenesis.

Experimental models in all the above cases have shown that the pharmacological inhibition of ATX attenuated further development of pathological and inflammatory conditions, thus revealing the high importance of ATX as a potential drug target. The enzyme activity of lysophospholipase D (LysoPLD) of ATX and consequently the degree of its inhibition, is performed by various methods which are analyzed below.

### TOOS Activity Assay.

TOOS Assay is based to the ATX activity to catalytically cleave the Lysophosphatidylcholine (LPC) to lysophosphatidic acid (LPA) and choline. The liberated choline is oxidized by choline oxidase to betaine and hydrogen peroxide (H₂O₂). The latter in the presence of Horse Radish peroxidase (HRP) reacts with N-ethyl -N-(2-hydroxy-3-sulfopropyl)-3-methylaniline (TOOS reagent) and 4-amino-2,3-dimethyl-1-phenyl-3-pyrazolin-5-one (4-AAP) to form a pink quinoneimine dye with a maximum absorbance at 555 nm.

### FS-3 Activity Assay

Plasma ATX activity is determined by measuring the cleavage of the ATX-specific fluorogenic substrate FS-3 (a double-labeled analogue of LPC). Assay was conducted in a final volume of 100 µl in the presence of a 20 µl of 5X reaction solution (700 mM NaCl, 25 mM KCI, 5 mM MgCl₂ and 250 mM Tris, pH 8.0), 20 µl FS-3 (final concentration 2.5 µM) and 60 µl plasma (dilution 1:5 in PBS). Reactions were incubated at 37 °C on a Tecan Infinite 200 microplate reader (Tecan Trading AG, Switzerland) and the fluorescence increase is measured at 528 nm.

### Amplex Red Activity Assay

In a similar approach to TOOS Activity Assay, this widely used method detects the ATX ability to hydrolyze LPC using a test that monitors choline release using Amplex Red fluorescent reagent (10 mM), choline oxidase (0.2 U/mL) and Radish Peroxidase (HRP - 2 U/mL). All reactions were conducted with 2 nM pure ATX and 50 µM LPC (16:0 or 18:0) at 37 °C. Inhibitors (3 mM) were dissolved in dimethyl sulfoxide (DMSO) at concentrations from 0.001 to 5 µmol/L. Fluorescence values were measured on a fluorescent plate reader (Tecan Infinite 200). IC₅₀ values were determined using the values from two independent experiments, using the sigmoid dose-response curve (PrismH software) from the equation f = y0 + a / (1 + exp (- (x-x₀) / b) given from SigmaPlot 11.0.

In this frame, the resolution of the crystal structure of ATX allowed scientists to design new drugs as potential inhibitors of ATX. The ATX sequence in rats is 93% similar to the corresponding one for humans and has been widely used in *in vitro* inhibition studies. The crystal structure of ATX has a central catalytic phosphodiesterase domain and a nuclease-like domain located at the C-terminus and a hydrophobic amino-terminal region consisting of two somatomedine B-like domains [Nakanaga, K., et al. Autotaxin-an LPA producing enzyme with diverse functions. Journal of Biochemistry, 2010, 148(1): 13-24]. The role of these areas is not entirely clear however, recent studies have shown that all three centers are potentially active for catalytic action. In this context, various small organic molecules have been proposed as potential inhibitors of ATX activity through their association with these centers. The structures of these molecules are selected through a process of controlling their interaction with the various crystalline forms of ATX and their optimization often involves the application of *in silico* procedures.

Based on a virtual screening of a library including more than 14400 chemical molecules, it was surprisingly found that the molecule N-[2-(4-nitrophenyl)-2,5-dihydro-4H-thieno-[3,4-c]-pyrazol-3-yl-naphthaleneacetamide (A1) can act as an ATX inhibitor presenting an IC₅₀ value of 8.6 µM, when examined in established experimental *in vitro* protocols.

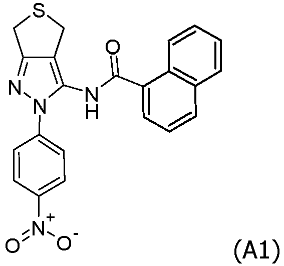

Document EP1698335A1 refers to chemical compounds with the general structure (B), said compounds are effective in treating diseases associated with mitochondrial benzodiazepine receptors, like diseases related to the central nervous or respiratory or digestive system caused by stress, and may include sleep disturbance, insomnia and depression, furthermore, chronic obstructive pulmonary disease, furthermore irritable bowel syndrome or peptic ulcer.

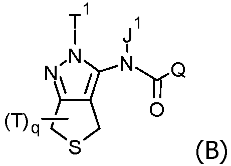

Documents WO2012/158413 and WO2014/078322 presents the compound 2-phenyl 5-oxo-4,6-dihydro-2H-thieno[3,4-c]pyrazol-3-yl phenyloxyacetamide (C) as an intermediate for the synthesis of substituted analogues of urea and thiourea exhibiting TrkA (Tropomyocin-related Kinase - Trk) inhibition.

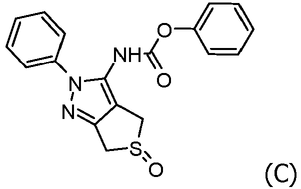

However, there is no reference in the prior art that these compounds can act as ATX inhibitors and in the treatment of pathological and inflammatory conditions caused by this activity. US 2010/087415 A1 discloses compounds and pharmaceutical compositions containing such compounds, which modulate the activity of store-operated calcium (SOC) channels. The article "Isoform-selective regulation of mammalian cryptochromes" by Miller et al. (published in Nature Chemical Biology, Vol. 16, No. 6, 30 March 2020) discloses phenotypic screenings of circadian clock modulators, and identified KL101 and TH301 that selectively stabilize CRY1 and CRY2, respectively. US 2008/081833 A1 discloses pyrazole derivatives and their use as modulators of nicotinic acetylcholine receptors. US 2010/075993 A1 discloses flavivirus replication inhibitors of WNV and other flaviviruses, such as, for example, JEV, SLEV, AV, KV, JV, CV, YV, TBEV, DENV-1, DENV-2, DENV-3, DENV-4, YFV and MVEV. The article "Development of Autotaxin Inhibitors: An Overview of the Patent and Primary Literature : Miniperspective" by Castagna et al. (published in Journal of Medicinal Chemistry, Vol. 59, No. 12, 28 January 2016) discloses a concise overview of the development of novel ATX inhibitors, including the disclosure of the first ATX clinical trial data. The article "Trial registration numbers" by Maher et al. (published in BMJ Open Respiratory Research, Vol. 6, No. 1, 1 May 2019) discloses two phase III, randomised, placebo-controlled studies of GLPG1690 (ziritaxestat), a novel autotaxin inhibitor, for use in the treatment of idiopathic pulmonary fibrosis. EP 1 698 335 A1 discloses a preventive and/or therapeutic agent for disease in which mitochondrial benzodiazepine receptor participates.

The current invention is defined in the claims and described in the following:
The invention as defined in the claims refers to a pharmaceutical compound or pharmaceutically acceptable salt thereof, for use in the treatment of pulmonary fibrosis, pulmonary allograft fibrosis, hepatic fibrosis, renal fibrosis, cardiac fibrosis, pneumonia, COVID-19 disease, bacterial infection, rheumatoid arthritis, neurodegenerative diseases, diabetes or diabetes mellitus, metabolic syndrome diseases and cancer, due to autotaxin inhibition, having the chemical formula (A), 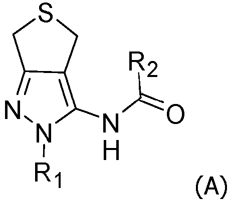 wherein:
- R₁ is selected from a heterocyclic group, or a heteroaromatic group, or 4-bromophenyl- or 4-nitrophenyl-groups,
- R₂ is selected from piperidino-, morpholino-, pyridino-, pyridazino-, pyrimidino-, pyrazino-, 3,5-dichlorophenyl-, naphthaleno-, 1-methoxy-1-(4-bromophenyl)-n-butyl-, (2,2,2-trifluoro-1-methoxy-1-phenyl)ethyl-groups.

In the pharmaceutical compound , R₁ is preferably selected from piperidine, piperidazine, piperazine, morpholine, pyridine, pyridazine, pyrimidine, pyrazine, oxazine, or an aromatic group which is selected from 4-bromophenyl, 4-carboxyphenyl- or 4-nitrophenyl-groups, more preferably selected from pyridine, pyrazine, 4-nitrophenyl-, or 4-bromophenyl-groups.

In the pharmaceutical composition , R₂ is selected from piperidino-, morpholino-, pyridino-, pyridazino-, pyrimidino-, pyrazino-, 3,5-dichlorophenyl-, naphthaleno-, 1-methoxy-1-(4-bromophenyl)-n-butyl-, (2,2,2-trifluoro-1methoxy-1-phenyl)ethyl-groups as defined in the claims, more preferably from 3,5-dichlorophenyl-, naphthaleno- and (2,2,2-trifluoro-1-methoxy-1phenyl)ethyl-groups.

In the pharmaceutical compound R₁ can be pyrazine and R₂ can be naphthaleno-group.

In the pharmaceutical compound R₁ can be 4-bromophenyl-group and R₂ can be (2,2,2-trifluoro-1-methoxy-1-phenyl)ethyl-group.

The invention further refers to a pharmaceutical composition for use complementary or stand-alone in the treatment of pulmonary fibrosis, pulmonary allograft fibrosis, hepatic fibrosis, renal fibrosis, cardiac fibrosis, pneumonia, COVID-19 disease, bacterial infection, rheumatoid arthritis, neurodegenerative diseases, diabetes or diabetes mellitus, metabolic syndrome diseases and cancer, due to autotaxin inhibition, the composition comprising a compound as defined in the claims, furthermore, comprising one or more pharmaceutically acceptable excipients

The pharmaceutical composition can be for complementary use in the treatment of cancer, due to autotaxin inhibition.

It was surprisingly found that N-[2-(4-nitrophenyl)-2,5-dihydro-4H-thieno-[3,4-c]-pyrazol-3-yl-naphthaleneacetamide (A1) has a clear indication in the treatment of pulmonary fibrosis, by inhibiting the mechanism of the enzymatic action of autotaxin.

It was surprisingly found that N-[2-(4-nitrophenyl)-2,5-dihydro-4H-thieno-[3,4-c]-pyrazol-3-yl-naphthaleneacetamide (A1) has a clear indication in the treatment of hepatic fibrosis, by inhibiting the mechanism of the enzymatic action of autotaxin.

It was surprisingly found that N-[2-(4-nitrophenyl)-2,5-dihydro-4H-thieno-[3,4-c]-pyrazol-3-yl-naphthaleneacetamide (A1) has a clear indication in the treatment of renal fibrosis, by inhibiting the mechanism of the enzymatic action of autotaxin.

It was surprisingly found that N-[2-(4-nitrophenyl)-2,5-dihydro-4H-thieno-[3,4-c]-pyrazol-3-yl-naphthaleneacetamide (A1) has a clear indication in the treatment of cardiac fibrosis, by inhibiting the mechanism of the enzymatic action of autotaxin.

It was surprisingly found that N-[2-(4-nitrophenyl)-2,5-dihydro-4H-thieno-[3,4-c]-pyrazol-3-yl-naphthaleneacetamide (A1) has a clear indication in the treatment of pneumonia, by inhibiting the mechanism of the enzymatic action of autotaxin.

It was surprisingly found that N-[2-(4-nitrophenyl)-2,5-dihydro-4H-thieno-[3,4-c]-pyrazol-3-yl-naphthaleneacetamide (A1) has a clear indication in the treatment of pulmonary allograft fibrosis, by inhibiting the mechanism of the enzymatic action of autotaxin.

It was surprisingly found that N-[2-(4-nitrophenyl)-2,5-dihydro-4H-thieno-[3,4-c]-pyrazol-3-yl-naphthaleneacetamide (A1) has a clear indication in the treatment of bacterial infection, by inhibiting the mechanism of the enzymatic action of autotaxin.

It was surprisingly found that N-[2-(4-nitrophenyl)-2,5-dihydro-4H-thieno-[3,4-c]-pyrazol-3-yl-naphthaleneacetamide (A1) has a clear indication in the treatment of rheumatoid arthritis, by inhibiting the mechanism of the enzymatic action of autotaxin.

It was surprisingly found that N-[2-(4-nitrophenyl)-2,5-dihydro-4H-thieno-[3,4-c]-pyrazol-3-yl-naphthaleneacetamide (A1) has a clear indication in the treatment of neurodegenerating diseases, by inhibiting the mechanism of the enzymatic action of autotaxin.

It was surprisingly found that N-[2-(4-nitrophenyl)-2,5-dihydro-4H-thieno-[3,4-c]-pyrazol-3-yl-naphthaleneacetamide (A1) has a clear indication in the treatment of metabolic syndrome diseases, by inhibiting the mechanism of the enzymatic action of autotaxin.

According to the present invention, substituents with molecular diversity in terms of their electronic, stereochemical and physicochemical properties (lipophilicity, polar surface area) may be incorporated at positions 2 and 3 of the thieno-pyrazole ring and exhibit similar drug activity N-[2-4-nitrophenyl)-2,5-dihydro-4H-thieno-[3,4-c]-pyrazol-3-yl-naphthaleneacetamide by inhibiting the enzymatic activity of ATX.

According to the present invention, the substituent located at position 2 of the pyrazole ring is selected from a heterocyclic group, or a heteroaromatic group, or 4-bromophenyl- or 4-nitrophenyl-groups as defined in the claims.

According to the present invention, the substituent located at position 2 of the pyrazole ring can be selected from a heterocyclic group or a heteroaromatic group, which may independently carry one or more substituents selected from halogen, nitro-, methoxy-, and carboxylic-group.

According to the present invention, the substituent located at position 2 of the pyrazole ring can be selected from a heterocyclic group, which is selected from piperidine, piperidazine, piperazine, which may independently carry one or more substituents selected from halogen, nitro-, methoxy-, and carboxylic-group.

According to the present invention, the substituent located at position 2 of the pyrazole ring can be selected from a heteroaromatic group which is selected from pyridine, pyridazine, pyrimidine, pyrazine, which may independently carry one or more substituents selected from halogen, nitro-, methoxy-, and carboxylic-group.

According to the present invention, the substituent located at position 2 of the pyrazole ring can be selected 4-bromophenyl- or 4-nitrophenyl-groups.

According to the present invention, the substituents which are linked via an amide bond at position 3 of the pyrazole ring are selected from piperidino-, morpholino-, pyridino-, pyridazino-, pyrimidino-, pyrazino-, 3,5-dichlorophenyl-, naphthaleno-, 1-methoxy-1-(4-bromophenyl)-n-butyl-, (2,2,2-trifluoro-1methoxy-1- phenyl)ethyl-groups as defined in the claims.

In a preferred embodiment, the pharmaceutical compound (A) of the current invention carries a 4-bromophenyl-group in position R₁ and a naphthaleno-group in position R₂.

In a preferred embodiment, the pharmaceutical compound (A) of the current invention carries a 2-pyrazino-group in position R₁ and a naphthaleno-group in position R₂.

In a preferred embodiment, the pharmaceutical compound (A) of the current invention carries a 4-pyridino-group in position R₁ and a naphthaleno-group in position R₂.

In a preferred embodiment, the pharmaceutical compound (A) of the current invention carries a 4-nitrophenyl-group in position R₁ and a naphthaleno-group in position R₂.

In a preferred embodiment, the pharmaceutical compound (A) of the current invention carries a 4-bromophenyl-group in position R₁ and a (2,2,2-trifluoro-1-methoxy-1-phenyl)ethyl-group in position R₂.

In a preferred embodiment, the pharmaceutical compound (A) of the current invention carries a 4-bromophenyl-group in position R₁ and a 3,5-dichlorophenyl-group in position R₂.

According to the present invention structural analogues of the pharmaceutical compound A1 can be produced following synthetic procedures known in the prior art. According to the synthetic procedure presented in **Scheme 1,** 4-cyano-3-oxo-tetrahydrothiophene **1** is produced by a Michael-Dieckmann reaction starting from methyl thioacetate and acrylonitrile. Reaction of **1** with hydrochloride salts of substituted hydrazines **2,** in ethanol under reflux, leads to 3-amino-2-(R₁-substituted)-2,5-dihydro-4H-thieno-[3,4-c]-pyrazoles **3.** Reaction of intermediate **3** with acyl chloride **4** in the presence of a base leads to derivative (A) which exhibits pharmacological activity by inhibiting the enzymatic activity of ATX.

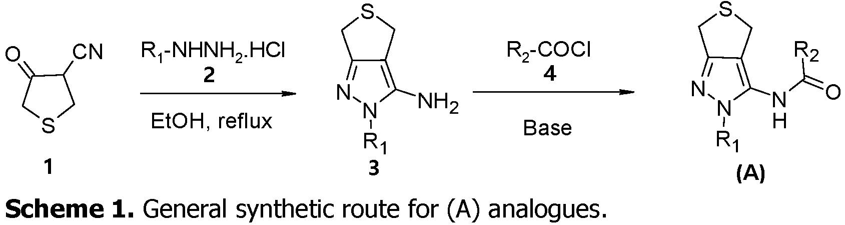

According to the present invention some illustrative non-limiting examples of compounds with chemical structure (A) and pharmacological action as ATX inhibitors, result from the combination of any substituent R₁ of the first column with any substituent R₂ of the second column of Table 1.

**Table 1.** Disclosure of combinations of derivatives with general formula (A) with pharmacological action due to inhibition of ATX.

| **Substituent R₁** | **Substituent R₂** |
|---|---|
| 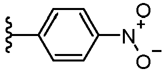 | 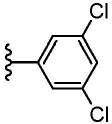 |
| 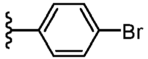 | 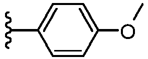 |
| 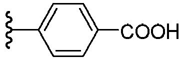 | 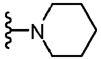 |
| 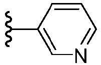 | 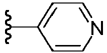 |
| 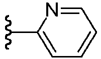 | 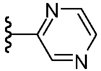 |
| 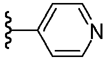 | 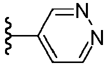 |
| 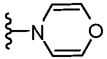 | 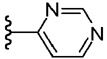 |
| 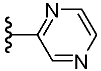 | 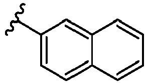 |
| 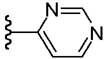 | 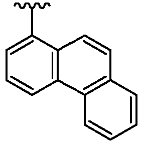 |
| 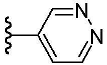 | 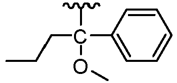 |
| 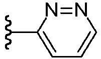 | 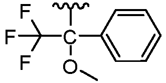 |

According to the present invention, the pharmaceutical compounds of chemical structure (A) can be mixed with suitable pharmaceutically acceptable excipients and formed in several pharmaceutical dosage forms, in an appropriate amount to achieve their therapeutic effect.

According to the present invention, chemical compounds of the formula (A) are suitable for use either as monotherapy or as adjuvant therapy in pulmonary fibrosis, furthermore in allograft pulmonary fibrosis, sarcoidosis and nonspecific interstitial pneumonia (NSIP), in acute pneumonia, in ventilator-induced pneumonia, in severe acute respiratory syndrome, in pneumonia, in COVID-19 disease, in hepatic fibrosis, including non-alcoholic fatty liver disease, non-alcoholic hepatitis C and hepatitis C or furthermore, in renal fibrosis, cardiac fibrosis, ocular fibrosis, bacterial infection, rheumatoid arthritis and other rheumatic diseases, in neurological and neurodegenerative diseases, in metabolic syndrome diseases, including diabetes, and diabetes mellitus and all cancer cases including cancer of the lung, breast, prostate, bone, in melanoma and in metastases.

The current invention is further described in the following representative examples.

### Example 1. Synthesis and activity evaluation of derivative (A4)

The synthesis of derivative (A4) is illustrated in **Scheme 2.** A solution of 4-cyano-3-oxo-tetrahydrothiophene (10 mmol) and 4-bromophenylhydrazine hydrochloride **5** (11 mmol) in 40 mL ethanol, is heated to reflux for 4 hours. Subsequently, the reaction mixture was evaporated in vacuo and the residue was redissolved in 50 mL H₂O and cooled to 0 °C. The cold solution was alkalized with a 5% sodium hydroxide solution up to pH 10, where crude **6** precipitated. The crude solid was recrystallized from a mixture of ethanol/water to give in pure form 3-amino-2-(4-bromophenyl)-2,5-dihydro-4H-thieno-[3,4-c]-pyrazole **6.** In the next step 3-amino-2-(4-bromophenyl)-2,5-dihydro-4H-thieno-[3,4-c]-pyrazole **6** (5 mmol) dissolved in anhydrous dichloromethane (5 mL) and triethylamine (6 mmol) was added, followed by gradual addition of naphthalene acyl chloride **7,** and the resulting solution is stirred at room temperature for 24 hours. The reaction mixture was extracted first with 5% hydrochloric acid solution and then with saturated sodium bicarbonate solution, the organic phase was separated and dried with sodium sulfate. Evaporation of the solvent followed by purification in the aid of column chromatography (hexane: dichloromethane: isopropanol) leads to the final derivative (A4) as white solid. Yield: 61%. ¹H-NMR (DMSO-d₆): 3.98 (s, 2H), 4.03 (s, 2H), 7.54-7.62 (m, 5H), 7.73 (d, *J =* 8.7 Hz, 3H), 8.02 (t, *J =* 7.9 Hz, 2H), 8.09 (d, *J* = 8.2 Hz, 1H), 10.71 (s, 1H). MS: (M+1) = 451.0.

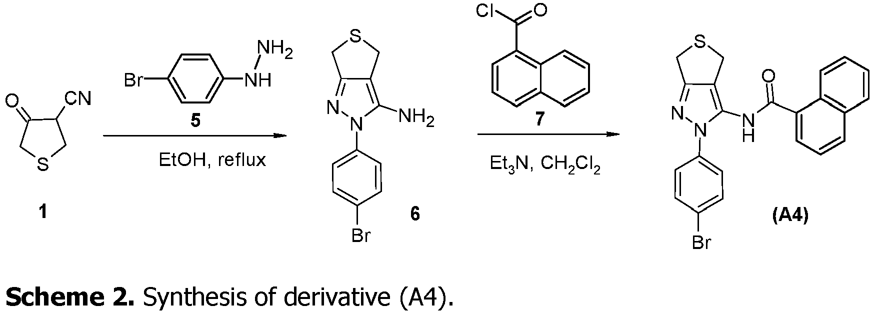

The ATX inhibitory activity for compound (A4) was assessed *in vitro* using the Amplex Red activity assay.

Briefly, 2 µL of concentration from 0,001 to 5 µmol/L of derivative (A4) in DMSO was incubated with 50 µL ATX 8 nM (up to a final concentration of 2 nM and 1 nM for human ATX) and 48 µL of a buffer solution (50 mM Tris-Cl of pH 8.0 and 5 mM CaCl₂) for 15 minutes at 37 °C. Subsequently, 50 µL of a buffer solution containing 200 µM LPC 16:0 (final concentration 50 µM) were added to the reaction mixture followed by incubation for another 30 minutes at 37 °C. Finally, 50 µL of working solution containing 200 µM Amplex Red reagent and choline oxidase (0.2 U/mL) κα 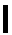 HRP (2 U/mL), in 50 mM Tris-HCl at pH 8.0 and 5 mM CaCl₂, was added to begin the reaction. The reaction was monitored at 37 °C, in a fluorescence plate reader (Tecan Infinite 200) using excitation at 530 nm and reading at 590 nm, every five minutes for 30 minutes at 37°C. The IC₅₀ value was calculated from two independent experiments, using the sigmoid dose-response curve (PrismH software) from the equation f=y0+a/(1+exp(-(x-x0)/b) given from SigmaPlot 11.0.

The IC₅₀ value for derivative (A4) was calculated as 12.7 µM for the inhibition of mouse ATX and 3.5 µM for human ATX inhibition.

### Example 2. Synthesis and activity evaluation of derivative (A14).

The synthesis of derivative (A14) is illustrated in **Scheme 3.** A solution containing 4-cyano-3-oxo-tetrahydrothiophene (10 mmol) and 2-hydrazinopyrazine **8** (11 mmol) in 4M hydrochloric acid in dioxane (50 mL), in 40 mL ethanol, is heated to reflux for 72 hours. Subsequently, the reaction mixture was evaporated in vacuo and the residue was redissolved in H₂O followed by extraction with diethylether. Then the aqueous phase was treated with a 5% solution of sodium hydroxide till ph 10 followed by extraction with ethyl acetate. The organic phase is dried, filtered and the solvent evaporated under vacuum. In the next step, 3-amino-2-(4-pyrazino)-2,5-dihydro-4H-thieno-[3,4-c]-pyrazole **9** (5 mmol) dissolved in anhydrous dioxane (5 mL) and triethylamine (6 mmol) was added, followed by gradual addition of naphthalene acyl chloride **7,** and the resulting solution is stirred under reflux for 48 hours. Evaporation of the solvent followed by recrystallization with ethyl acetate leads to the final derivative (A14) as a black solid. Yield: 11%. ¹H-NMR (DMSO-d₆): 3.70 (s, 2H), 3,86 (s, 2H), 7.58-7.64 (m, 3H), 7.77 (d, *J* = 8.7 Hz, 3H), 7,82 (m, 1H), 7.97 (m, 1H), 8.15 (d, *J* = 8.2 Hz, 1H), 8.45 (s, 1H), 10.81 (s, 1H). MS: (M+1) = 374.1.

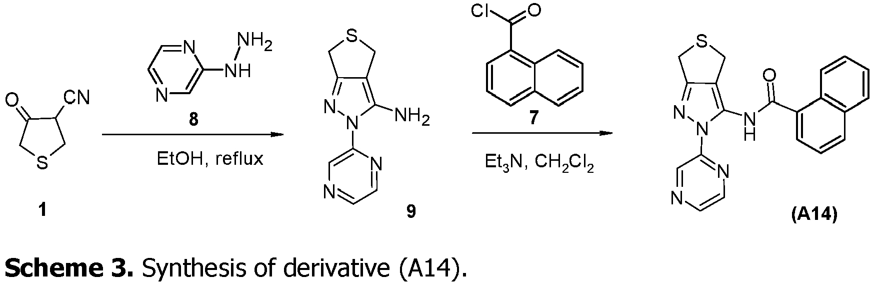

The ATX inhibitory activity for compound (A14) was assessed *in vitro* using the Amplex Red activity assay.

Briefly, 2 µL of concentration from 0,001 to 5 µmol/L of derivative (A4) in DMSO was incubated with 50 µL ATX 8 nM (up to a final concentration of 2 nM and 1 nM for human ATX) and 48 µL of a buffer solution (50 mM Tris-Cl of pH 8.0 and 5 mM CaCl₂) for 15 minutes at 37 °C. Subsequently, 50 µL of a buffer solution containing 200 µM LPC 16:0 (final concentration 50 µM) was added to the reaction mixture followed by incubation for another 30 minutes at 37 °C. Finally, 50 µL of working solution containing 200 µM Amplex Red reagent and choline oxidase (0.2 U/mL) κα 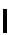 HRP (2 U/mL), in 50 mM Tris-HCl at pH 8.0 and 5 mM CaCl₂, was added to begin the reaction. The reaction was monitored at 37 °C, in a fluorescence plate reader (Tecan Infinite 200) using excitation at 530 nm and reading at 590 nm, every five minutes for 30 minutes at 37°C. The IC₅₀ value was calculated from two independent experiments, using the sigmoid dose-response curve (PrismH software) from the equation f=y0+a/(1+exp(-(x-x0)/b) given from SigmaPlot 11.0.

The IC₅₀ value for derivative (A14) was calculated as 2.5 µM for the inhibition of mouse ATX and 1.3 µM for human ATX inhibition.

### Example 3. Synthesis and activity evaluation of derivative (A16)

The synthesis of derivative (A16) is illustrated in **Scheme 4.** A solution of 4-cyano-3-oxo-tetrahydrothiophene (10 mmol) and 4-hydrazinopyridine hydrochloride **10** (11 mmol) in 40 mL ethanol, is heated to reflux for 24 hours. Subsequently, the reaction mixture was evaporated in vacuo and the residue was redissolved in 50 mL H₂O and cooled to 0 °C. The cold solution was alkalized with a 5% sodium hydroxide solution up to pH 10, where 3-amino-2-(2-pyridino)-2,5-dihydro-4H-thieno-[3,4-c]-pyrazole **11** precipitated, filtered, washed with water, dried under vacuum and used in the next step without any further purification. In the next step 3-amino-2-(2-pyridino)-2,5-dihydro-4H-thieno-[3,4-c]-pyrazole **11** (5 mmol) was dissolved in anhydrous dioxane (5 mL) in the aid of triethylamine (6 mmol), followed by gradual addition of naphthalene acyl chloride **7,** and the resulting solution is stirred under reflux for 18 hours. The reaction mixture was extracted first with 5% hydrochloric acid solution and then with a saturated solution of sodium bicarbonate, the organic phase was separated and dried with sodium sulfate. Evaporation of the solvent followed by purification in the aid of column chromatography (eluent: hexane:ethyl acetate) leads to the final derivative (A16) as a brownish solid. Yield: 37%. ¹H-NMR (DMSO-d₆): 3.99 (s, 2H), 4.07 (s, 2H), 7,70 (d, *J =* 7.0 Hz, 2H), 7.85 (d, *J =* 6.7 Hz, 1H), 8.02 (d, *J =* 7.0 Hz, 2H), 8.11-8.17 (m, 3H), 8.68 (d, *J =* 6.2 Hz, 2H), 8.86 (d, *J =* 8.6 Hz, 1H), 10.91 (s, 1H). MS: (M+1) = 451.0. MS: (M+1) = 373.1.

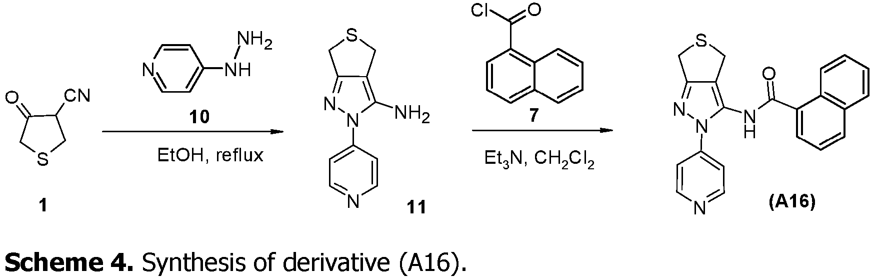

The ATX inhibitory activity for compound (A16) was assessed *in vitro* using the Amplex Red activity assay.

Briefly, 2 µL of concentration from 0,001 to 5 µmol/L of derivative (A4) in DMSO was incubated with 50 µL ATX 8 nM (up to a final concentration of 2 nM and 1 nM for human ATX) and 48 µL of a buffer solution (50 mM Tris-Cl of pH 8.0 and 5 mM CaCl₂) for 15 minutes at 37 °C. Subsequently, 50 µL of a buffer solution containing 200 µM LPC 16:0 (final concentration 50 µM) was added to the reaction mixture followed by incubation for another 30 minutes at 37 °C. Finally, 50 µL of working solution containing 200 µM Amplex Red reagent and choline oxidase (0.2 U/mL) κα 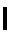 HRP (2 U/mL), in 50 mM Tris-HCl at pH 8.0 and 5 mM CaCl₂, was added to begin the reaction. The reaction was monitored at 37 °C, in a fluorescence plate reader (Tecan Infinite 200) using excitation at 530 nm and reading at 590 nm, every five minutes for 30 minutes at 37°C. The IC₅₀ value was calculated from two independent experiments, using the sigmoid dose-response curve (PrismH software) from the equation f=y0+a/(1+exp(-(x-x0)/b) given from SigmaPlot 11.0.

The IC₅₀ value for derivative (A16) was calculated as 7.5 µM for the inhibition of human ATX.

### Example 4. Synthesis and activity evaluation of derivative (A17)

The synthesis of derivative (A16) is illustrated in **Scheme 5.** A solution of 4-cyano-3-oxo-tetrahydrothiophene (10 mmol) and 4-bromophenylhydrazine hydrochloride **5** (11 mmol) in 40 mL ethanol, is heated to reflux for 4 hours. Subsequently, the reaction mixture was evaporated in vacuo and the residue was redissolved in 50 mL H₂O and cooled to 0 °C. The cold solution was alkalized with a 5% sodium hydroxide solution up to pH 10, where crude **6** precipitated. The crude solid was recrystallized from a mixture of ethanol/water to give in pure form 3-amino-2-(4-bromophenyl)-2,5-dihydro-4H-thieno-[3,4-c]-pyrazole **6.** In the next step 3-amino-2-(4-bromophenyl)-2,5-dihydro-4H-thieno-[3,4-c]-pyrazole **6** (5 mmol) was dissolved in anhydrous pyridine (125 mmol), followed by gradual addition of 3,3,3-trifluoro-2-methoxy-2-phenylpropanoyl chloride **11,** and the resulting solution is stirred at room temperature for 24 hours. The reaction mixture was extracted first with 5% hydrochloric acid solution and then with saturated sodium bicarbonate solution, the organic phase was separated and dried with sodium sulfate. Evaporation of the solvent followed by purification in the aid of column chromatography (hexane: dichloromethane: isopropanol) leads to the final derivative (A17) as white solid. Yield: 74%. ¹H-NMR (DMSO-d₆): 3.51 (s, 3H), 3.81 (m, 3H), 4.00 (s, 2H), 7.25-7.29 (m, 2H), 7.43-7.52 (m, 5H), 7.53-7.55 (m, 2H), 10.48 (s, 1H). MS: (M+1) = 513.2.

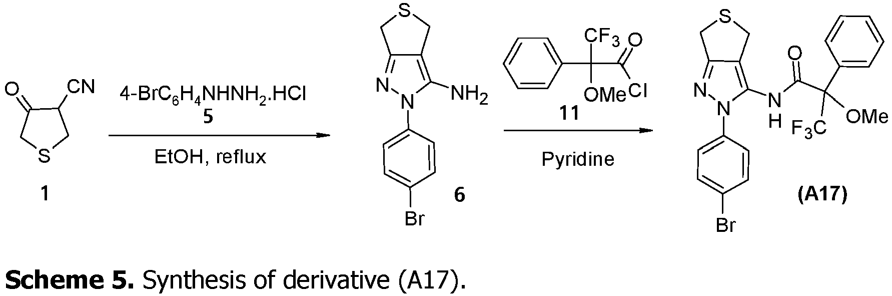

The ATX inhibitory activity for compound (A17) was assessed *in vitro* using the Amplex Red activity assay.

Briefly, 2 µL of concentration from 0,001 to 5 µmol/L of derivative (A17) in DMSO was incubated with 50 µL ATX 8 nM up to a final concentration of 1 nM for human ATX and 48 µL of a buffer solution (50 mM Tris-Cl of pH 8.0 and 5 mM CaCl₂) for 15 minutes at 37 °C. Subsequently, 50 µL of a buffer solution containing 200 µM LPC 16:0 (final concentration 50 µM) was added to the reaction mixture followed by incubation for another 30 minutes at 37 °C. Finally, 50 µL of working solution containing 200 µM Amplex Red reagent and choline oxidase (0.2 U/mL) κα 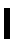 HRP (2 U/mL), in 50 mM Tris-HCl at pH 8.0 and 5 mM CaCl₂, was added to begin the reaction. The reaction was monitored at 37 °C, in a fluorescence plate reader (Tecan Infinite 200) using excitation at 530 nm and reading at 590 nm, every five minutes for 30 minutes at 37°C. The IC₅₀ value was calculated from two independent experiments, using the sigmoid dose-response curve (PrismH software) from the equation f=y0+a/(1+exp(-(x-x0)/b) given from SigmaPlot 11.0.

The IC₅₀ value for derivative (A17) was calculated as 2.0 µM for the inhibition of human ATX.

### Example 5. Table 2 refers to representative, non-limiting derivatives of formula (A) with IC₅₀ in the range of µM, as regards the inhibitory activity of ATX.

**Table 2. Disclosure of combinations of derivatives with ATX inhibition activity (mATX) in the range of µM.**

| Structure | **R₁** | **R₂** | IC₅₀ (µM) (in mATX 2 nM) |
|---|---|---|---|
| **(A1)** | 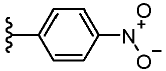 | 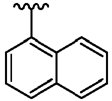 | 8.6 |
| **(A4)** | 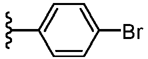 | 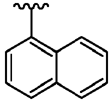 | 12.7 |
| **(A14)** | 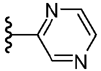 | 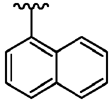 | 2.5 |
| **(A15)** | 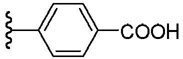 | 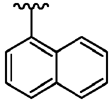 | 59.28 |
| **(A16)** | 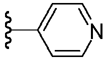 | 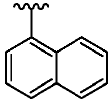 | 7.5* |
| **(A17)** | 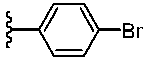 | 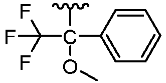 | 2.0* |
| **(A18)** | 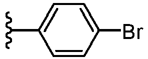 | 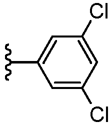 | 2.5* |

| | | | |
|---|---|---|---|
| * The evaluation performed in human ATX (hATX) 1 nM. | | | |

## Claims

1. A pharmaceutical compound or pharmaceutically acceptable salt thereof, for use in the treatment of pulmonary fibrosis, pulmonary allograft fibrosis, hepatic fibrosis, renal fibrosis, cardiac fibrosis, pneumonia, COVID-19 disease, bacterial infection, rheumatoid arthritis, neurodegenerative diseases, diabetes or diabetes mellitus, metabolic syndrome diseases and cancer, due to autotaxin inhibition, having the chemical formula (A), 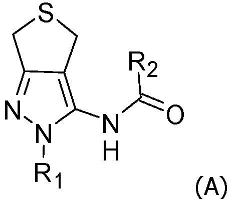 wherein:
- R₁ is selected from a heterocyclic group, or a heteroaromatic group, or 4-bromophenyl- or 4-nitrophenyl-groups,
- R₂ is selected from piperidino-, morpholino-, pyridino-, pyridazino-, pyrimidino-, pyrazino-, 3,5-dichlorophenyl-, naphthaleno-, 1-methoxy-1-(4-bromophenyl)-n-butyl-, (2,2,2-trifluoro-1-methoxy-1phenyl)ethyl-groups.

2. The pharmaceutical compound for use according to claim 1, wherein R₁ is selected from pyridine, pyrazine, 4-nitrophenyl-, or 4-bromophenyl-groups.

3. The pharmaceutical compound for use according to claim 1, wherein R₂ is selected from 3,5-dichlorophenyl-, naphthaleno- and (2,2,2-trifluoro-1-methoxy-1phenyl)ethyl-groups.

4. The pharmaceutical compound for use according to any of the preceding claims, wherein R₁ is pyrazine and R₂ is naphthaleno-group.

5. The pharmaceutical compound for use according to any of the preceding claims, wherein R₁ is a 4-bromophenyl-group and R₂ is a (2,2,2-trifluoro-1-methoxyy-1-phenyl)ethyl-group.

6. The pharmaceutical compound or pharmaceutically acceptable salt thereof for use according to claim 1, wherein the pharmaceutical compound has the chemical formula (A1):

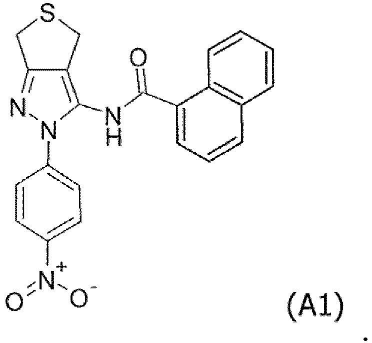

7. A pharmaceutical composition for use complementary or stand-alone in the treatment of pulmonary fibrosis, pulmonary allograft fibrosis, hepatic fibrosis, renal fibrosis, cardiac fibrosis, pneumonia, COVID-19 disease, bacterial infection, rheumatoid arthritis, neurodegenerative diseases, diabetes or diabetes mellitus, metabolic syndrome diseases and cancer, due to autotaxin inhibition, the composition comprising a compound as defined in any of the preceding claims, furthermore, comprising one or more pharmaceutically acceptable excipients.

8. The pharmaceutical composition for use according to claim 7, for complementary use in the treatment of cancer, due to autotaxin inhibition.

## Patentansprüche

1. Pharmazeutische Verbindung oder pharmazeutisch verträgliches Salz davon zur Verwendung bei der Behandlung von Lungenfibrose, Lungenallotransplantatfibrose, Leberfibrose, Nierenfibrose, Herzfibrose, Lungenentzündung, COVID-19-Erkrankung, bakterieller Infektion, rheumatoider Arthritis, neurodegenerativen Erkrankungen, Diabetes oder Diabetes mellitus, Metabolisches-Syndrom-Erkrankungen und Krebs aufgrund der Hemmung von Autotaxin, mit der chemischen Formel (A), 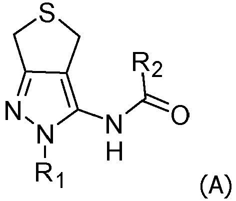 wobei:
- R₁ ausgewählt ist aus einer heterocyclischen Gruppe oder einer heteroaromatischen Gruppe, oder 4-bromphenyl- oder 4-nitrophenyl-Gruppen,
- R₂ ausgewählt ist aus Piperidino-, Morpholino-, Pyridino-, Pyridazino-, Pyrimidino-, Pyrazino-, 3,5-Dichlorphenyl-, Naphthaleno-, 1-Methoxy-1-(4-bromphenyl)-n-butyl-, (2,2,2-Trifluor-1-methoxy-1-phenyl)ethyl-Gruppen.

2. Pharmazeutische Verbindung zur Verwendung gemäß Anspruch 1, wobei R₁ ausgewählt ist aus Pyridin-, Pyrazin-, 4-Nitrophenyl- oder 4-Bromphenylgruppen.

3. Pharmazeutische Verbindung zur Verwendung gemäß Anspruch 1, wobei R₂ ausgewählt ist aus 3,5-Dichlorphenyl-, Naphthalen- und (2,2,2-trifluor-1-methoxy-1-phenyl)ethyl-Gruppen.

4. Pharmazeutische Verbindung zur Verwendung gemäß einem beliebigen der vorhergehenden Ansprüche, wobei R₁ Pyrazin ist und R₂ eine Naphthalingruppe ist.

5. Pharmazeutische Verbindung zur Verwendung gemäß einem beliebigen der vorhergehenden Ansprüche, wobei R₁ eine 4-Bromphenylgruppe und R₂ eine (2,2,2-Trifluor-1-methoxy-1-phenyl)ethylgruppe ist.

6. Pharmazeutische Verbindung oder pharmazeutisch verträgliches Salz davon zur Verwendung gemäß Anspruch 1, wobei die pharmazeutische Verbindung die chemische Formel (A1) aufweist:

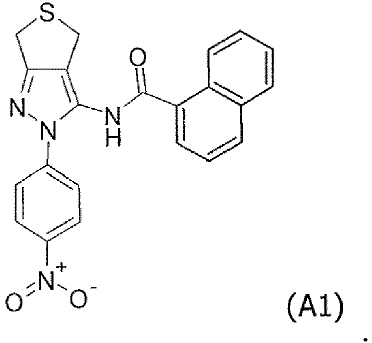

7. Pharmazeutische Zusammensetzung zur ergänzenden oder alleinigen Verwendung bei der Behandlung von Lungenfibrose, Lungenallotransplantatfibrose, Leberfibrose, Nierenfibrose, Herzfibrose, Lungenentzündung, COVID-19-Erkrankung, bakterieller Infektion, rheumatoider Arthritis, neurodegenerativen Erkrankungen, Diabetes oder Diabetes mellitus, Metabolisches-Syndrom-Erkrankungen und Krebs aufgrund der Hemmung von Autotaxin, wobei die Zusammensetzung eine Verbindung gemäß einem beliebigen der vorhergehenden Ansprüche umfasst und darüber hinaus einen oder mehrere pharmazeutisch verträgliche Hilfsstoffe umfasst.

8. Pharmazeutische Zusammensetzung zur Verwendung gemäß Anspruch 7, zur ergänzenden Verwendung bei der Behandlung von Krebs aufgrund von Autotaxin-Hemmung.

## Revendications

1. Composé pharmaceutique ou sel pharmaceutiquement acceptable de celui-ci, pour une utilisation dans le traitement de la fibrose pulmonaire, de la fibrose d'allogreffe pulmonaire, de la fibrose hépatique, de la fibrose rénale, de la fibrose cardiaque, de la pneumonie, de la COVID-19, d'une infection bactérienne, de la polyarthrite rhumatoïde, de maladies neurodégénératives, du diabète ou du diabète sucré, de syndromes métaboliques et du cancer, dus à l'inhibition de l'autotaxine, répondant à la formule chimique (A) : 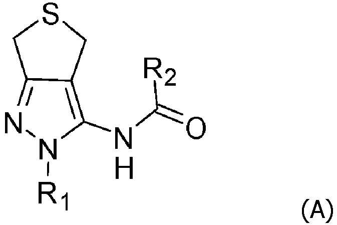 dans laquelle :
- R₁ est choisi parmi un groupe hétérocyclique ou un groupe hétéroaromatique ou les groupes 4-bromophényl- ou 4-nitrophényl- ;
- R₂ est choisi parmi les groupes pipéridino-, morpholino-, pyridino-, pyridazino-, pyrimidino-, pyrazino-, 3,5-dichlorophényl-, naphtaléno-, 1-méthoxy-1-(4-bromophényl)-n-butyl-, (2,2,2-trifluoro-1-méthoxy-1-phényl)éthyl-.

2. Composé pharmaceutique pour une utilisation selon la revendication 1, dans lequel R₁ est choisi parmi les groupes pyridine, pyrazine, 4-nitrophényl- ou 4-bromophényl-.

3. Composé pharmaceutique pour une utilisation selon la revendication 1, dans lequel R₂ est choisi parmi les groupes 3,5-dichlorophényl-, naphtaléno- et (2,2,2-tri-fluoro-1-méthoxy-1-phényl)éthyl-.

4. Composé pharmaceutique pour une utilisation selon l'une quelconque des revendications précédentes, dans lequel R₁ est la pyrazine et R₂ est le groupe naphtaléno-.

5. Composé pharmaceutique pour une utilisation selon l'une quelconque des revendications précédentes, dans lequel R₁ est un groupe 4-bromophényl- et R₂ est un groupe (2,2,2-trifluoro-1-méthoxy-1-phényl)éthyl-.

6. Composé pharmaceutique ou sel pharmaceutiquement acceptable de celui-ci, pour une utilisation selon la revendication 1, dans lequel le composé pharmaceutique répond à la formule chimique (A1) :

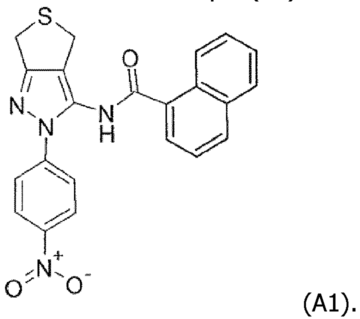

7. Composition pharmaceutique pour une utilisation en complément ou seule dans le traitement de la fibrose pulmonaire, de la fibrose d'allogreffe pulmonaire, de la fibrose hépatique, de la fibrose rénale, de la fibrose cardiaque, de la pneumonie, de la COVID-19, d'une infection bactérienne, de la polyarthrite rhumatoïde, de maladies neurodégénératives, du diabète ou du diabète sucré, de syndromes métaboliques et du cancer, dus à l'inhibition de l'autotaxine, la composition comprenant un composé tel que défini selon l'une quelconque des revendications précédentes, en outre, comprenant un ou plusieurs excipients pharmaceutiquement acceptables.

8. Composition pharmaceutique pour une utilisation selon la revendication 7, pour une utilisation en complément dans le traitement du cancer, du fait de l'inhibition de l'autotaxine.
